# EUROPÄISCHE PATENTSCHRIFT

(11) **EP 3 367 972 B1**
(45) Veröffentlichungstag und Bekanntmachung des Hinweises auf die Patenterteilung: **02.12.2020**
(21) Anmeldenummer: 16794545.0
(22) Anmeldetag: 27.10.2016
(51) Int. Cl.: A61F 2/28, A61F 2/30, A61B 34/10

(54) **AUTOMATISIERTE GENERIERUNG VON KNOCHENBEHANDLUNGSMITTELN**
AUTOMATED GENERATION OF BONE TREATMENT AGENT
GENERATION AUTOMATISEE DES AGENT DE TRAITEMENT OSSEUX

(30) Priorität: 27.10.2015 DE 102015118318
(43) Veröffentlichungstag der Anmeldung: 05.09.2018
(73) Patentinhaber: Karl Leibinger Medizintechnik GmbH & Co. KG, 78570 Mühlheim/Donau (DE)
(72) Erfinder: LEIBINGER, Christian, 78570 Mühlheim (DE); HÖLLER, Wolfgang, 4320 Perg (AT); MARTIN, Michael, 79199 Kirchzarten (DE)
(74) Vertreter: Winter, Brandl, Fürniss, Hübner, Röss, Kaiser, Polte - Partnerschaft mbB
(86) Internationale Anmeldenummer: PCT/EP2016/075923
(87) Internationale Veröffentlichungsnummer: WO 2017/072227

(56) Entgegenhaltungen:
- EP-A1- 0 255 797
- WO-A1-01/85040
- WO-A1-2008/109751
- WO-A2-01/77988
- DE-A1- 19 922 279
- FR-A1- 2 999 071
- US-A- 4 936 862

## Beschreibung

Die Erfindung betrifft ein Verfahren zum Herstellen von Knochenbehandlungsmitteln beispielsweise orthognathen Osteosynthesen und/oder Schablonen.

Benachbarte Verfahren sind bspw. aus den US-Patenten US 8 855 389 B1 und US 2014/0094924 A1 bekannt. Die US 8 855 389 B1 offenbart eine computerimplementierte Methode zum Verwenden einer Finite-Elemente-Methode für Knochenimplantat-Systeme. Dabei wird auch auf eine Bibliothek mit vorkonstruierten Implantatsdaten zugegriffen. Diese Daten werden aber auf einen intakten Knochen appliziert / gemorphed. In der US 2014/0094924 A1 wird hingegen auf ein Spiegelbild eines intakten / unbeschädigten kontralateralen Knochens zurückgegriffen. Weiterer Stand der Technik ist aus der US 2011/0151 400 A1, der US 4 936 862 A, der EP 0 255 797 A1 und der WO 2008/109571 A1 bekannt.

Ferner ist Stand der Technik aus der DE 199 22 279 A1 bekannt. Hiermit ist eine Generierung patientenspezifischer Implantate durch unterschiedliche Methoden offenbart. In einer Methode wird ein virtuelles Implantatmodell über ein 3D-Referenzmodell generiert. Dazu wird anhand eines Vergleiches von spezifischen Merkmalen des zuvor erfassten 3D-Patientenmodells in einer Referenzdatenbank eine Auswahl an ähnlichen Modellen unter Berücksichtigung mathematischer, funktioneller, medizinischer und ästhetischer Gesichtspunkte getroffen. Aus dieser Auswahl wird letztlich, vorzugsweise unter besonderer medizinischer Begutachtung, das 3D-Referenzmodell selektiert. Durch Überlagerung des 3D-Referenzmodells und des 3D-Patientenmodells entsteht ein virtuelles 3D-Patientenmodell, aus dem wiederum das virtuelle Implantatmodell am Rechner generiert wird.

Bei den bestehenden Verfahren zum Herstellen von Knochenbehandlungsmitteln, seien es nun Implantate, oder Knochentrenn- / Knochenschnittschablonen, ist die Qualität nicht zufriedenstellend hoch. Es soll hier eine Verbesserung zur Verfügung gestellt werden, wobei insbesondere die Passgenauigkeit des Knochenbehandlungsmittels für den zu behandelnden Patienten weiter zu verbessern ist. Ferner soll es ermöglicht werden, dass schneller, kostengünstiger und einfacher auf die individuellen Patienten angepasste Knochenbehandlungsmittel hergestellt und zur Verfügung gestellt werden können. Auch soll eine höhere Planungssicherheit gewährleistet werden. Die Planung soll ferner einfacher werden. Letztlich soll auch die Bedienerfreundlichkeit eines solchen Verfahrens verbessert werden.

Diese Aufgabe wird erfindungsgemäß durch den Gegenstand des Anspruchs 1 gelöst. Gegenüber der DE 199 22 279 A1 ergibt sich nach Anspruch 1 somit der Unterschied, dass sich die hinzugezogenen 3D-Daten des Referenzpatienten aus 3D-Daten unterschiedlicher einzelner Patienten anhand einer Mittelwertbildung und/oder eines statistischen Modells zusammensetzen und nicht, wie mit der DE 199 22 279 A1 umgesetzt, durch ein Patientenmodell eines einzigen Patienten, das der zu behandelnden Stelle am ähnlichsten ist, gebildet werden. Ferner ist mit der DE 199 22 279 A1 auch nicht offenbart, dass weiterhin ein bereits durch Standard-Mittelwerte erzeugtes, in einer Datenbank hinterlegtes Durchschnittsimplantat, das anhand von Konstruktionspunkten in dem statistischen Modell verankert ist, an der entsprechenden zuvor rekonstruierten zu behandelnden Stelle automatisch platziert wird, und wobei anschließend eine Anpassung einer dem Knochen zugewandten Oberfläche des Durchschnittsimplantats durchgeführt wird. Der Anspruch 1 führt folglich zu einer deutlichen Steigerung der erzielten Passgenauigkeit des gefertigten Implantates mit der Stelle des zu behandelnden Patienten.

Mit anderen Worten umfasst der Gegenstand des Anspruchs 1 ein Verfahren zum Herstellen von Knochenbehandlungsmitteln, bei dem unterschiedliche Schritte eingesetzt werden. So soll in einem ersten Schritt (ursprüngliche) 3D-Daten eines Knochens oder eines Knochenabschnitts eines spezifischen, zu behandelnden Patienten erfasst / abgerufen / genutzt werden, wobei innerhalb des Knochens oder des Knochenabschnitts eine zu behandelnde Stelle vorhanden ist. Diese Stelle ist üblicherweise ein Defekt oder eine Knochenmaterialfehlstelle. Diese (ursprünglichen) 3D-Daten eines Knochens oder eines Knochenabschnitts des spezifischen, zu behandelnden Patienten gehen bspw. zurück auf einen Datenerfassungsschritt, etwa mittels CT-, MRT-, MRI, DICOM- oder ähnliche Verfahren und Vorrichtungen. In einem dritten, rekonstruierenden Schritt werden die beiden Datensätze so miteinander verknüpft, dass ein Ergänzen oder Vervollständigen von 3D-Daten für die Rekonstruktion der zu behandelnden Stelle stattfindet. Hierbei ist ein Trimmen der ursprünglichen 3D-Daten oder der erhaltenen ergänzten Daten auf Basis der 3D-Daten des ausgewählten Referenzpatienten enthalten oder kann enthalten sein, wodurch rekonstruierte und getrimmte 3D-Daten etwa zum Überbrücken oder Ergänzen einer Knochenmaterialfehlstelle beim zu behandelnden Patienten erhalten werden. Dadurch wird eine wesentliche Verbesserung gegenüber bisherigen Verfahren erreicht. Die Daten des "Referenzpatienten" können insbesondere einen Datensatz betreffen, der aus unterschiedlichen einzelnen Patienten zusammengesetzt wurde. Hierbei können bspw. Mittelwertbildungen, Medianbildungen und/oder andere/ähnliche Algorithmen Verwendung finden. Unter dem "Referenzpatienten" muss also nicht zwingend eine "Einzelperson" zu verstehen sein, kann aber so sein. Es bietet sich an, einen "künstlichen" "Referenzpatienten" aus bestehenden Datensätzen zusammenzusetzen. Es findet letztlich also ein statistisches Modell Einsatz. Unter einem Patienten wird eine lebende oder eine tote Person bzw. Tier und/oder Teile davon verstanden. Ein Spiegelungsschritt wird genutzt, um mittels eines Überlagerns von 3D-Daten des spezifischen, zu behandelnden Patienten, auf die betroffene Stelle kombinierte 3D-Daten der zu behandelnden Stelle zu erhalten, wobei die überlagerten 3D-Daten ihren Ursprung auf einer spiegelsymmetrisch anderen Seite des Patienten finden, und zwar an einer dem Knochen oder dem Knochenabschnitt korrespondierenden Stelle. Während schon das Hinzuziehen von statistischen Daten, nämlich der Daten, die von einem oder mehreren Referenzpatienten zur Verfügung gestellt wurden, eine Verbesserung zeigt, wird diese Verbesserung durch das Nutzen eines Spiegelungsschrittes und Nutzen der gespiegelten Daten der gesunden Seite des spezifischen, individuellen Patienten, welcher zu behandeln ist, deutlich weiter verbessert. Somit ist also auch ein Schritt des Hinzuziehens von 3D-Daten vorgesehen, wobei diese 3D-Daten dem Knochen oder dem Knochenabschnitt mit der zu behandelnden Stelle (allerdings auf der gesunden Seite) entsprechen, also ihren Ursprung auf einer zur behandelnden Seite spiegelsymmetrischen Seite finden.

Vorteilhafte Ausführungsformen sind in den Unteransprüchen beansprucht und werden nachfolgend näher erläutert.

Es ist von Vorteil, wenn das Ergebnis zumindest der drei Schritte für die Planung der Operation genutzt wird.

Es ist auch von Vorteil, wenn die drei Schritte des zur Verfügungstellens der ursprünglichen 3D-Daten des zu behandelnden Patienten, des Hinzuziehens der 3D-Daten des Referenzpatienten und dem Schritt des Ergänzens, nacheinander oder zumindest teilweise parallel ablaufen. So lassen sich Planabschnitte von 5 Minuten bis 10 Minuten einhalten, und sogar eine gesamte Fertigung von ≤ 12 Stunden erreichen, wenn vor Ort gefertigt wird oder ≤ 48 Stunden, wenn ein Medizintechnik-Unternehmen an physisch anderer Stelle eingeschalten wird.

Wenn nach dem dritten Schritt in einem vierten Schritt das Knochenbehandlungsmittel, nach Art eines Implantats oder einer Knochenschnittschablone gefertigt wird, ist recht schnell ein am Knochen zu befestigendes Bauteil zur Verfügung stellbar.

Es hat sich auch als vorteilhaft herausgestellt, wenn in einem Vorbereitungsschritt die ursprünglichen 3D-Daten des Patienten und/oder die 3D-Daten eines oder mehrerer Referenzpatienten in eine bspw. webbasierte Datenbank gespielt werden und/oder dieser entnommen werden.

Ein vorteilhaftes Ausführungsbeispiel ist auch dadurch gekennzeichnet, dass vor dem Spiegelungsschritt und/oder nach dem ersten Schritt eine rechnergestützte 2D- oder 3D-Visualisierung durchgeführt wird. Die Bedienerfreundlichkeit wird dadurch erhöht.

Damit eine Identifizierung der einzelnen Knochen bzw. Knochenfragmente effizient und einfach durchgeführt werden kann, ist es von Vorteil, wenn vor oder nach dem Spiegelungsschritt, vorzugsweise nach dem Schritt des Visualisierens, definierte Knochenmarkierungspunkte, etwa nach Art von Landmarks, "Landmarken" oder Markierungen, ausgewählt werden / sind. Damit nicht nur vorhandene Knochen verbessert werden können, sondern auch tatsächlich fehlendes Material ersetzt werden kann, ist es von Vorteil, wenn eine Knochenmaterialfehlstelle des zu behandelnden Patienten nach Art eines Loches geschlossen oder überbrückt oder aufgefüllt wird. Dadurch lässt sich der Einsatzbereich des Verfahrens deutlich erweitern. Natürlich ist es auch möglich, das Knochenbehandlungsmittel so einzusetzen, dass es nach dessen Befestigung am Knochen / dem Knochenabschnitt, als führende und/oder lenkende Einrichtung zum Perforieren, Schneiden oder Durchlöchern des Knochens dient.

Dem Patienten kann schneller als bisher geholfen werden, wenn vor dem vierten Schritt, nämlich dem Fertigen, in einem Erzeugungsschritt 3D-Daten und/oder Fertigungsdaten zur Ansteuerung von Fertigungsmaschinen, etwa NC- oder CNC-Daten erzeugt werden und vorteilhafterweise diese NC- oder CNC-Daten direkt oder indirekt in eine Fertigungsvorrichtung, wie eine Steuervorrichtung einer Fräs-, Dreh-, Sinter- oder Schweißanlage gefüttert werden. Urformende, umformende, insbesondere spanabhebende und/oder additive Fertigungsverfahren lassen sich dann schnell und effizient einsetzen. Besonders vorteilhaft ist der der Einsatz von Rapid-Prototyping-Techniken, wie 3D-Druck- / 3D-Print-Techniken, insbesondere solche, die ein *.3mf-Datenformat nutzen. Es sollten neben Geometrieinformationen auch Fertigungsinformationen zur additiven und/oder spanabhebenden Fertigung beinhaltet sein.

Ferner ist es von Vorteil, wenn vorzugsweise direkt nach dem dritten Schritt und/oder vor dem vierten Schritt ein Modellierungsschritt zum Erzielen von Oberflächen, Achsen, Lokalisationen und/oder Deviationsfaktoren durchgeführt wird.

Es ist zweckmäßig, wenn dem vierten Schritt vorgelagert oder anstelle des vierten Schrittes ein Operationsplanungsschritt durchgeführt wird.

Ein vorteilhaftes Ausführungsbeispiel ist auch dadurch gekennzeichnet, dass die 3D-Daten des zu behandelnden Patienten und/oder die 3D-Daten des oder der Referenzpatienten in/auf einer Datenbank eines Krankenhauses oder einem I-Cloud-Server (oder einer ähnlichen Einrichtung) oder einer Datenbank eines Medizintechnik-Unternehmens gespeichert sind. Sowohl krankenhausinterne, krankenhausexterne, als auch all-verfügbare-Daten sind dann einsetzbar. Insbesondere durch eine webbasierte Lösung, wird die Akzeptanz des Verfahrens verbessert und der Einsatz erleichtert.

Wenn die 3D-Daten des oder der Referenzpatienten Auswahlkriterien, wie Raucher- / Nichtraucher-, Geschlechts-, Alter-, Größe-, Beruf-, Ethnie- und/oder Konstitutionsphysiologie-Informationen enthalten, wird das Auswählen der jeweils (individuell) passenden Daten zum Rekonstruieren des Knochens erleichtert. Für die Konstitutionsphysiologie-Informationen ist die Einordnung nach Kretschmer geeignet, obwohl dessen Einordnung kontrovers diskutiert wird.

Die Erfindung betrifft auch eine Vorrichtung zum Durchführen eines Planungs- und/oder Herstellverfahrens, wobei Mittel enthalten / eingerichtet und vorbereitet sind, die das erfindungsgemäße Verfahren durchführen.

Eine Weiterbildung besteht darin, dass ein Computer umfasst / enthalten ist, der vorbereitet und eingerichtet ist, die Schritte des Verfahrens automatisch durchzuführen. Eine Interaktion mit einem Bedienpersonal wird somit auf ein Minimum beschränkt.

Eine erfindungsgemäße Verwendung besteht darin, Unregelmäßigkeiten in einem Knochen einzusetzen und somit eine bessere Diagnose zu erhalten.

Mit anderen Worten wird nun ein Verfahren oder Prozess beschrieben, bei dem unter Nutzung statistischer Formmodelle eine Oberfläche und/oder ein Volumen generiert wird, auf der und/oder in dem die Implantat-Rekonstruktion und die Schablonen für Osteotomie in einer Datenbank hinterlegt sind. Hierdurch können Knochenbehandlungsschritte und/oder Knochenbehandlungsmittel für/auf jedes Individuum automatisch angepasst und errechnet werden. Die Knochenbehandlungsmittel lassen sich auch individuell angepasst und besonders zeitnah herstellen.

Statistische Modelle von anatomischen Regionen bieten sich für die medizinische Planung an. Hierbei handelt es sich um virtuelle Modelle, die es erlauben, anhand existierender individueller Form-Informationen, fehlende oder defekte Bereiche zu ergänzen oder zu ersetzen.

Es hat sich gezeigt, dass die statistischen Modelle zur Rekonstruktion des knöchernen Stützapparates des Menschen eine höhere Genauigkeit ermöglichen / aufweisen, als eine einfache / singuläre Spiegelung der gesunden auf die erkrankte Seite. So besteht ein großer Vorteil darin, dass bei der automatisierten Rekonstruktion des pathologisch oder traumatologisch veränderten Knochens lediglich eine Ausrichtung anhand von Punkten oder Oberflächen auf ortsständigen Knochen erforderlich ist, um das statistische Formmodell anzuwenden und eine Rekonstruktion unabhängig von aufwändigeren Segmentationsverfahren zu erhalten. Zudem ist die Art und Qualität der vorhandenen 3D-Bildinformationen des Individuums nun unabhängig auf das Ergebnis der Rekonstruktion durch das statistische Modell. Dies bedeutet auch, dass das Vorhandensein von Artefakten, etwa zurückgehend auf Metallschrauben, bedingend Unschärfebereiche in bildgebenden Diagnoseverfahren, herausgerechnet werden können und so entfernt werden können.

Kombiniert man das statistische Modell mit Implantat-Konstruktionen, so bedeutet dies, dass diese an das jeweilige Individuum automatisiert angepasst werden können. Durch die Auswahl von typischen Frakturlokalisationen, können so z.B. automatisiert individuelle Implantate erzeugt werden. Weiterhin besteht eine Idee darin, die Informationen der einzelnen Rekonstruktionen zu sammeln, um so eine Implantat-Optimierung für standardisierte Durchschnittsimplantate zu erhalten.

Das gleiche Prinzip lässt sich auch auf die so genannten "Cutting guides" anwenden. "Cutting guides" werden benötigt, errechnete Osteotomien am Knochen durchzuführen. So wird z.B. bei einer Unterkieferrekonstruktion, bei der ein Knochentransplantat aus dem Wadenbein eingesetzt werden soll, im Vorfeld errechnet, wie der gehobene Knochen geschnitten werden muss, damit man die anatomische Form des Unterkiefers nachkonstruieren kann. Hinterlegt man diese Defekte in einer Datenbank, so können die "Cutting guides" automatisiert errechnet werden. Zudem kann durch ein solches Verfahren, die zusätzliche Röntgenbelastung der Spenderregion in Zukunft wegfallen, wenn das statistische Modell diese Informationen als Durchschnittswert automatisiert liefern kann, wovon momentan auszugehen ist.

Die Prozesskette zum Herstellen von Implantaten sieht dabei wie folgt aus:
1. Datenerfassung (CT, MRT, Ultraschall, statistisches Raster (Geschlecht, Alter, Größe, Beruf, ...))
2. Selektion der Region und/oder des Implantats durch Punkte oder Flächen
3. Anwendung eines statistischen Modells auf die selektierte Region
4. Deformation des Implantats auf die ihm zugewiesene Region
5. Export der fertigen Implantat-Konstruktionsdatei

Die Prozesskette für "Cutting Guide" lässt sich wie folgt charakterisieren:
1. Datenerfassung
2 Selektion der zu rekonstruierenden Region
3. Anwendung eines statistischen Modells auf die selektierte Region
4. Selektion der Spenderregion und Errechnung der notwendigen Osteotomien
5. Darstellung der notwendigen Umstellungskorrektur und automatische Konstruktion des Cutting Guides
6. Export der fertigen Konstruktionsdatei

Es ergeben sich diverse Vorteile gegenüber anderen Verfahren. So wird keine Spiegelung der Seite zwingend genutzt. Hierdurch kann die individuelle Asymmetrie berücksichtigt werden. Ein neues Aufkonstruieren des Implantats ist nicht notwendig. Es können beliebig viele "Rohimplantate" hinterlegt werden. Diese sind nach Indikation und Operateur abrufbar. "Cutting guides" können im Operationsplanungsverfahren mit berechnet werden. Die Prozesskette verkürzt sich dadurch deutlich. Die notwendige Prüfung des Arztes entfällt, da diese in der gleichen Sitzung der Implantatgenerierung durch den Planenden erfolgt. Die WEB-basierte Applikation erlaubt die Planung schnell und effizient ohne zusätzliche Software. Die Software kann im selbstlernenden Modus die Implantate und die Oberflächen stetig verbessern. Eine Hinterlegung von "Normmaßen" und "Normachsen", um pathologische Veränderungen zu detektieren und die entsprechende Korrektur vorzuschlagen, wird möglich. Eine zusätzliche Röntgenaufnahme und eine damit verbundene Strahlenbelastung der Spenderregion kann entfallen.

Das besondere ist also, dass eine automatische Rekonstruktion der Knochenoberfläche mit 3D-Daten stattfindet, und zwar unter Nutzung vorhandener Daten des spezifischen, individuellen Patienten, die mit Daten aus einem statischen Modell ergänzt werden. Die Kombination der vorhandenen (Rest-)Daten des individuellen Patienten mit den ergänzenden 3D-Daten aus dem Statistikmodel führt daher zu einer zielgenauen Oberflächenrekonstruktion des zu behandelnden Knochens.

Das statistische Formenmodell dient der computer-assistierten Planung. Hierbei wird das Formenmodell in die entsprechende Planungssoftware integriert (z.B. als STL-Datensatz) und kann zur "virtuellen Rekonstruktion" in der chirurgischen Navigation verwendet werden. Der Vorteil dieses Verfahrens liegt darin, dass keine Spiegelung zur Rekonstruktion durchgeführt werden muss aber kann. Hierdurch können bilaterale (zweiseitige Defekte navigiert werden). Das gleichzeitige Mitführen der virtuellen Implantate erlaubt die präzise Kontrolle der chirurgischen Positionierung durch die Navigation.

Eine besondere Anwendung besteht auch darin, dass in standardisiertes Implantat für eine Region bereits "aufkonstruiert" wird. D.h., anhand von Standard-Mittelwerten wurde bereits ein "Durchschnittsimplantat" erzeugt. Dieses ist in einer Datenbank hinterlegt. Anhand der Konstruktionspunkte wird dieses im statistischen Modell verankert und an die entsprechende Stelle des Individuums automatisch platziert. In einem zweiten Schritt erfolgt dann die Oberflächenanpassung der Implantatfläche, die dem Knochen zugewandt ist. Die Konstruktionsdatei ändert sich beim Anpassen des statistischen Formenmodells an den individuellen Knochen.

Besonders ist es auch, wenn ein Standardimplantat auf die entsprechende Stelle des Knochens aufgelagert wird (best fit). Durch ein Trimmverfahren wird dabei Material zwischen der Oberfläche des Implantates, die dem Knochen zugewandt ist, und dem Knochen aufgefüllt.

Die Erfindung wird nachfolgend mit Hilfe einiger Figur näher erläutert. Es zeigen:
- Fig. 1: ein Ablaufdiagramm zum Durchführen eines erfindungsgemäßen Verfahrens,
- Fig. 2: den Ablauf des Remodellierens an einem Knochen,
- Fig. 3: die Lage eines zu behandelnden Bereiches an einem beispielhaften Schädel und
- Fig. 4: das Angebrachtsein von Knochenbefestigungsmitteln , nach Art eines Orbita-Implantats und eines Oberkieferimplantats.

Die Figuren sind lediglich schematischer Natur und dienen nur dem Verständnis der Erfindung. Die gleichen Elemente sind mit denselben Bezugszeichen versehen.

Die Erfindung bietet sich zum Einsatz in der Schädel- und Gesichtschirurgie an, kann aber letztlich an und/oder für jede knöcherne Struktur eines Menschen oder eines Säugetiers eingesetzt werden.

Bei einem erfindungsgemäßen Verfahren 1 gibt es einen ersten Schritt 2 des zum Verfügungstellens von ursprünglichen 3D-Daten eines Knochens oder eines Knochenabschnittes eines spezifischen, zu behandelnden Patienten. Danach gibt es einen zweiten Schritt 3, bei dem das Hinzuziehen von 3D-Daten eines nach vordefinierten Kriterien ausgewählten Referenzpatienten stattfindet, nämlich die 3D-Daten in einem vergleichbaren Bereich, welcher zur Behandlung ansteht, entnommen werden. In einem nachfolgenden dritten Schritt 4 erfolgt ein Ergänzen, U.U. umfassend ein Trimmen, der aus dem Schritt 2 und dem Schritt 3 kombinierten 3D-Daten, wobei in einem Teilschritt ein kombinieren der Daten stattfindet.

Zwischen dem ersten Schritt 2 und dem zweiten Schritt 3 kann auch ein Spiegelungsschritt 5 stattfinden. Bei diesem Spiegelungsschritt, werden zur Längsachse bzw. einer Symmetrieebene, die die Längsachse des Körpers enthält, gegenüberliegende 3D-Daten am kranken, zu behandelnden (spezifischen) Patienten an gesunder Stelle entnommen und den 3D-Daten der zu behandelnden, kranken Seite überlagert. Es ist empfehlenswert, diesen Schritt zu nutzen.

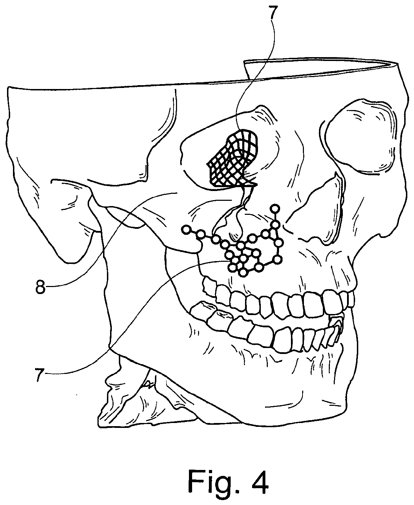

In einem vierten Schritt 6, der auch als Fertigungsschritt bezeichnet wird, wird ein Knochenbehandlungsmittel 7, etwa nach Art eines Implantats oder einer Knochenschnittschablone gefertigt. Damit ist auch ein "virtual surgical planning" möglich. Ein solches Knochenbehandlungsmittel 7, welches an einem Knochen 8 eines spezifischen, individuellen, zu behandelnden Patienten befestigt ist, ist in Fig. 4 dargestellt.

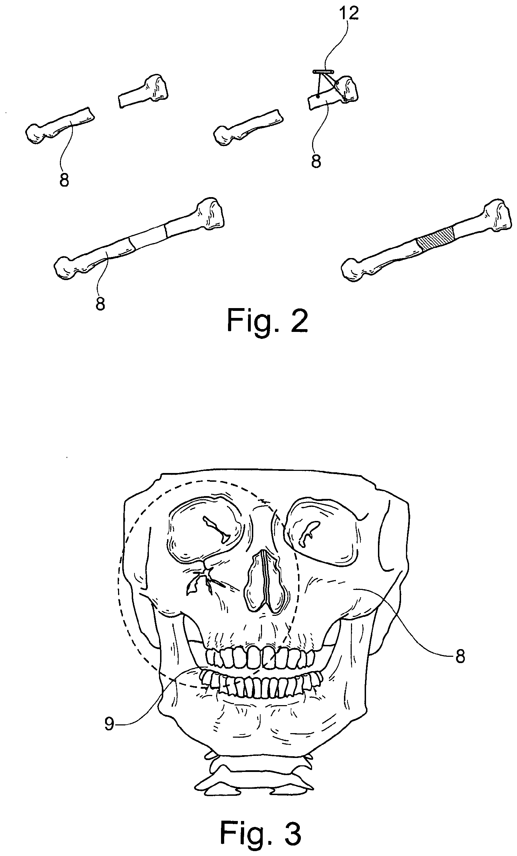

In Fig. 3 ist ein zu behandelnder Bereich 9 an einem einen Knochen 8 aufweisenden Schädel dargestellt. Während die Orbita dieses Schädels auf vom Patienten aus gesehen rechter Seite einen Defekt im Bereich des zu behandelnden Bereiches 9 aufweist, weist die Orbita auf vom Patienten aus gesehen linker Seite, keinen Defekt auf.

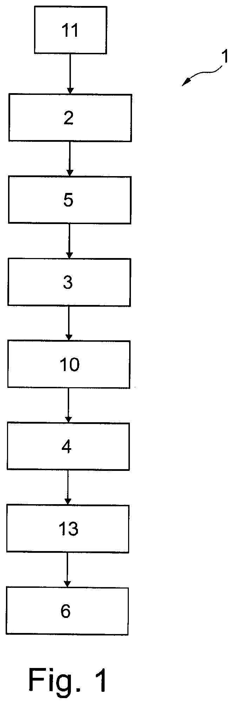

Die diesbezüglichen Daten, der gesunden Seite, werden in einem in Fig. 1 visualisierten Spiegelungsschritt 10 auf die defekte Stelle übertragen. Sie werden dort auf- / eingemorphed. Den bisherigen Schritten vorgelagert ist ein Vorbereitungsschritt 11, in dem die 3D-Daten des Patienten und/oder die 3D-Daten eines oder mehrerer Referenzpatienten, in eine lokale oder webbasierte Datenbank eingespielt werden bzw. dieser entnommen werden.

In Fig. 2 ist bildlich dargestellt, wie, von einem Knochendefekt ausgehend, Landmarken geschaffen werden, dann ein "adjustment" stattfindet, bei dem ein überlagertes Formmodell eingesetzt ist, was aber noch nicht adaptiert ist, um nachfolgend dann in einem Berechnungsschnitt ein statistisches Formmodell einzusetzen, um ein adaptiertes Modell mit ersetztem Knochendefekt zu erreichen. Markierungen 12, die die "Landmarks" bzw. Landmarken bilden, sind mit dem Bezugszeichen 12 gekennzeichnet.

Es geht also darum, dass bisher ausschließlich z.B. Schädeldefekte meist über Spiegelungen der gesunden Seite auf die defekte Seite rekonstruiert werden. Dies passt jedoch nur bedingt bzw. die Ergebnisse sind nicht ausreichend gut. Im vorliegenden Verfahren, wird eine Vielzahl von Schädelmodellen zu einem statistischen Modell ausgewertet. Aus dem statistischen Modell kann nun am defekten Schädel die defekte Stelle rekonstruiert werden.

Es ist ein Erzeugungsschritt 13 in dem erfindungsgemäßen Verfahren 1 eingesetzt.

### Bezugszeichen

- 1: Verfahren
- 2: erster Schritt (Daten zur Verfügungstellung)
- 3: zweiter Schritt (Hinzuziehen eines statistischen Modells)
- 4: dritter Schritt (Ergänzen evtl. zzgl. Trimmen)
- 5: Spiegelungsschritt
- 6: vierter Schritt / Fertigungsschritt
- 7: Knochenbehandlungsmittel
- 8: Knochen
- 9: zu behandelnder Bereich
- 10: Spiegelungsschritt
- 11: Vorbereitungsschritt
- 12: Markierung
- 13: Erzeugungsschritt

## Patentansprüche

1. Verfahren (1) zum Herstellen eines Implantats (7), mit einem ersten Schritt (2), in dem ursprüngliche 3D-Daten eines Knochens (8) oder eines Knochenabschnitts eines spezifischen, zu behandelnden Patienten zur Verfügung gestellt werden, wobei innerhalb des Knochens (8) oder des Knochenabschnitts eine zu behandelnde Stelle vorhanden ist, mit einem zweiten Schritt (3) des Hinzuziehens von 3D-Daten eines nach vordefinierten Kriterien ausgewählten Referenzpatienten, wobei die hinzugezogenen 3D-Daten des Referenzpatienten dem Knochen (8) oder dem Knochenabschnitt mit der zu behandelnden Stelle entsprechen und aus 3D-Daten unterschiedlicher einzelner Patienten anhand einer Mittelwertbildung und/oder eines statistischen Modells zusammengesetzt werden, und mit einem dritten, rekonstruierenden Schritt (4) zum Ergänzen oder Vervollständigen der aus dem ersten Schritt (2) und dem zweiten Schritt (3) kombinierten 3D-Daten für die Rekonstruktion der zu behandelnden Stelle,
wobei weiterhin ein bereits durch Standard-Mittelwerte erzeugtes, in einer Datenbank hinterlegtes Durchschnittsimplantat, das anhand von Konstruktionspunkten in dem statistischen Modell verankert ist, an der entsprechenden zuvor rekonstruierten zu behandelnden Stelle automatisch platziert wird, und wobei anschließend eine Anpassung einer dem Knochen zugewandten Oberfläche des Durchschnittsimplantats durchgeführt wird.

2. Verfahren (1) nach Anspruch 1, **dadurch gekennzeichnet, dass** ein Spiegelungsschritt (5) genutzt wird, in dem ein Überlagern von 3D-Daten des spezifischen, zu behandelnden Patienten, die ihren Ursprung auf einer spiegelsymmetrisch anderen Seite des Patienten finden, und zwar an einer zum Knochen (8) oder Knochenabschnitt korrespondierenden Stelle, zum Erhalten von kombinierten 3D-Daten durchgeführt wird.

3. Verfahren (1) nach Anspruch 1 oder 2, **dadurch gekennzeichnet, dass** die drei oder vier Schritte (2, 3, 4 und/oder 5) nacheinander oder parallel ablaufen.

4. Verfahren (1) nach einem der Ansprüche 1 bis 3, **dadurch gekennzeichnet, dass** nach dem dritten Schritt (4) in einem weiteren Schritt (6) das Knochenbehandlungsmittel (7) nach Art eines Implantats oder einer Knochenschnittschablone gefertigt wird.

5. Verfahren (1) nach Anspruch 4, **dadurch gekennzeichnet, dass** in einem Vorbereitungsschritt (11) die ursprünglichen 3D-Daten des Patienten und/oder die 3D-Daten eines oder mehrerer Referenzpatienten in eine Datenbank gespielt werden und/oder dieser entnommen werden.

6. Verfahren (1) nach einem der Ansprüche 2 bis 5, **dadurch gekennzeichnet, dass** vor dem Spiegelungsschritt (5) und/oder nach dem ersten Schritt (2) eine rechnergestützte 2D- oder 3D-Visualisierung durchgeführt wird.

7. Verfahren (1) nach einem der Ansprüche 2 bis 6, **dadurch gekennzeichnet, dass** vor oder nach dem Spiegelungsschritt (5) definierte Knochenmarkierungspunkte (12) ausgewählt werden / sind.

8. Verfahren (1) nach einem der Ansprüche 4 bis 7, **dadurch gekennzeichnet, dass** vor dem weiteren Schritt (6) in einem Erzeugungsschritt 3D-Daten und/oder Fertigungsdaten zur Ansteuerung von Fertigungsmaschinen erzeugt werden.

9. Verfahren (1) nach einem der vorhergehenden Ansprüche, **dadurch gekennzeichnet, dass** das Ergebnis zumindest der drei Schritte (2, 3, 4) für die Planung der Operation genutzt wird.

## Claims

1. A method (1) for producing an implant (7), with a first step (2) in which original 3D data of a bone (8) or a bone portion of a specific patient to be treated are provided, wherein a site to be treated is present inside the bone (8) or the bone portion, with a second step (3) of involving 3D data of a reference patient who has been selected according to predefined criteria, wherein the involved 3D data of the reference patient correspond to the bone (8) or the bone portion with the site to be treated and are composed of 3D data of various individual patients by means of formatting a mean value and/or a statistic model, and with a third and reconstructive step (4) for supplementing or completing the 3D data combined of the first step (2) and the second step (3) for the reconstruction of the site to be treated,
wherein a standardized implant, which was already generated by way of standard mean values and which is deposited in a database, is anchored in the statistical model by way of construction points and is automatically placed at the appropriate site to be treated which has been reconstructed beforehand, and wherein subsequently the surface of the standardized implant area facing the bone then is adapted.

2. The method (1) according to claim 1, **characterized in that** a mirroring step (5) is used in which 3D data of the specific patient to be treated which have their origin on a mirror-symmetrical other side of the patient are superposed, specifically at a site corresponding to the bone (8) or bone portion, in order to obtain the combined 3D data.

3. The method (1) according to claim 1 or 2, **characterized in that** the three or four steps (2, 3, 4 and/or 5) are run successively or in parallel.

4. The method (1) according to one of the claims 1 to 3, **characterized in that** after the third step (4) in a further step (6) the bone treatment means (7) is produced in the form of an implant or an osteotomy template.

5. The method (1) according to claim 4, **characterized in that** in a preparation step (11) the original 3D data of the patient and/or the 3D data of one or more reference patients are entered into a database and/or are gathered therefrom.

6. The method (1) according to one of the claims 2 to 5, **characterized in that** before the mirroring step (5) and/or after the first step (2) a computer-aided 2D or 3D visualization is carried out.

7. The method (1) according to one of the claims 2 to 6, **characterized in that** before or after the mirroring step (5) defined bone marker points (12) will be/ are selected.

8. The method (1) according to one of the claims 4 to 7, **characterized in that** prior to the further step (6) 3D data and/or manufacturing data for controlling manufacturing machines are generated in a generation step.

9. The method (1) according to one of the preceding claims, **characterized in that** the result of at least the three steps (2, 3, 4) is used for planning the operation.

## Revendications

1. Procédé (1) de fabrication d'un implant (7), comprenant une première étape (2), dans laquelle des données 3D initiales d'un os (8) ou d'une partie osseuse d'un patient spécifique à traiter sont mises à disposition, une zone à traiter existant dans l'os (8) ou la partie osseuse, comprenant une deuxième étape (3) de consultation des données 3D d'un patient de référence sélectionné selon des critères prédéfinis, les données 3D consultées du patient de référence correspondant à l'os (8) ou à la partie osseuse avec la zone à traiter et des patients différentes étant réunis à partir des données 3D à l'aide d'un calcul de la valeur moyenne et/ou d'un modèle statistique, et comprenant une troisième étape de reconstruction (4) pour ajouter ou compléter les données 3D combinées à partir de la première étape (2) et de la deuxième étape (3) pour la reconstruction de la zone à traiter,
en outre un implant moyen déjà produit par des valeurs moyennes standard, enregistré dans une banque de données, qui est ancré à l'aide de points de construction dans le modèle statistique, étant positionné automatiquement au niveau de la zone à traiter correspondante avant la reconstruction, et un ajustement étant ensuite réalisé d'une surface tournée vers l'os de l'implant moyen.

2. Procédé (1) selon la revendication 1, **caractérisé en ce qu'**une étape de symétrisation (5) est utilisée, dans laquelle une superposition de données 3D du patient spécifique à traiter est réalisée, qui proviennent d'un autre côté symétrique du patient, c.-à-d. au niveau d'une zone correspondante à l'os (8) ou à la partie osseuse, pour obtenir des données 3D combinées.

3. Procédé (1) selon la revendication 1 ou 2, **caractérisé en ce que** les trois ou quatre étapes (2, 3, 4 et/ou 5) se déroulent les unes après les autres ou en parallèle.

4. Procédé (1) selon l'une quelconque des revendications 1 à 3, **caractérisé en ce qu'**après la troisième étape (4) lors d'une autre étape (6) le moyen de traitement d'os (7) est fabriqué à la manière d'un implant ou d'un modèle de coupe osseuse.

5. Procédé (1) selon la revendication 4, **caractérisé en ce que** lors d'une étape de préparation (11) les données 3D initiales du patient et/ou les données 3D d'un ou plusieurs patients de référence sont représentées dans une base de données et/ou sont extraites de celle-ci.

6. Procédé (1) selon l'une quelconque des revendications 2 à 5, **caractérisé en ce qu'une** visualisation 2D ou 3D assistée par ordinateur est réalisée avant l'étape de symétrisation (5) et/ou après la première étape (2).

7. Procédé (1) selon l'une quelconque des revendications 2 à 6, **caractérisé en ce que** des points de marquage osseux (12) définis seront/sont sélectionnés avant ou après l'étape de symétrisation (5).

8. Procédé (1) selon l'une quelconque des revendications 4 à 7, **caractérisé en ce que** des données 3D et/ou des données de fabrication destinées à commander des machines de production sont générées avant l'étape suivante (6) selon une étape de génération.

9. Procédé (1) selon l'une quelconque des revendications précédentes, **caractérisé en ce que** le résultat d'au moins les trois étapes (2, 3, 4) est utilisé pour planifier l'intervention chirurgicale.
